# EUROPEAN PATENT APPLICATION

(11) **EP 0 753 523 A1**
(43) Date of publication of application: **15.01.1997**
(21) Application number: 95110706.9
(22) Date of filing: 10.07.1995
(51) Int. Cl.: C07F 9/38, A61K 31/66

(54) **Amino-substituted bisphosphonic acids**

(71) Applicant: GADOR S.A., Buenos Aires 1414 (AR); University of Leiden, NL-2330 RC Leiden (NL)
(72) Inventor: Papapoulos, Socrates, University of Leiden, NL-2300 RC Leiden (NL); van Beek, E.R., University of Leiden, NL-2300 RC Leiden (NL); Lowick, C.W.G.M., University of Leiden, NL-2300 RC Leiden (NL); Labriola, Rafael, BIOFARMA, AR-1619 Pilar (AR); Vecchioli, Adriana, BIOFARMA, AR-1619 Pilar (AR)
(74) Representative: Winkler, Andreas, Dr.

(57) **Abstract**

1-Aminoalkyliden-1,1-bisphosphonic acids of formula (B), or any salt thereof: 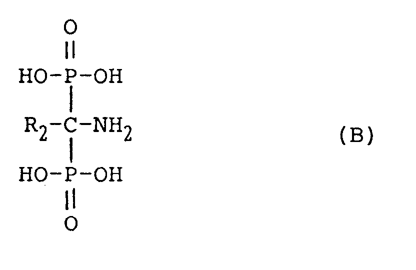 wherein R₂ is a straight-chain or branched aliphatic hydrocarbon radical of 1-9 carbon atoms, which is optionally substituted by one or more amino or aminoalkyl groups with the exception of a terminal aminoalkyl group 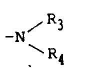 wherein R₃ is a straight-chain or branched, saturated or unsaturated aliphatic hydrocarbon radical of 1-9 carbon atoms, and R₄ is cyclohexyl or cyclohexylmethyl, benzyl or a straight-chain or branched, saturated or unsaturated aliphatic hydrocarbon radical of 4-18 carbon atoms, as a single substituent of R₂.

## Description

### Background of the Invention:

Bisphosphonates - synthetic compounds containing two phosphonate groups bound to a carbon and two additional groups R₁ and R₂, respectively (see formula (A) herebelow) - bind strongly to calcium crystals, inhibit their growth, suppress bone resorption and are used in the treatment of a variety of disorders of calcium and bone metabolism. It is generally considered that the "bone hook" (P-C-P with R₁) is responsible for the binding of these molecules to mineralized matrices, while, in turn, the R₂ group is primarily responsible for its effect on bone resorption.

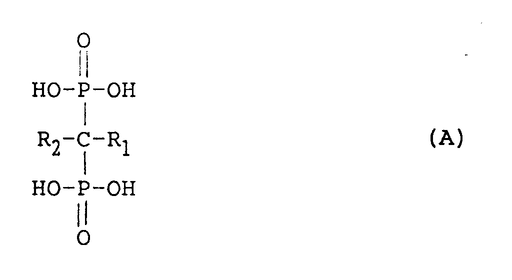

The availability of the R₁ and R₂ groups in the bisphosphonate structure allows numerous substitutions and development of a variety of analogs. U.S. Patent No. 3,962,432 discloses the use of aminoalkane bisphosphonic acids for the treatment of a variety of calcium disorders. U.S. Patent No. 5,006,329 discloses radioactive tagged compounds and a method for the treatment of metastatic bone cancer. U.S. Patent No. 5,039,819 discloses a bisphosphonate intermediate for preparing an antihypercalcemic agent. U.S. Patent No. 5,103,036 discloses a process for preparing 3-alkenylidene-1,1-bisphosphonates. U.S. patent No. 5,137,880 discloses bicyclic bisphosphonate compounds, pharmaceutical preparations containing the same, and methods for treating abnormal calcium and phosphate metabolism. U.S. Patent No. 5,205,253 discloses bisphosphonic acid derivatives, processes for their production and pharmaceutical preparations containing these compounds for the treatment of calcium metabolism. U.S. Patent No. 5,206,253 discloses a process for the production of bisphosphonic acid derivatives and pharmaceutical preparations containing these compounds. European Patent No. 541,037 A2 discloses 1,1-bisphosphonic acid compounds as squalene synthetase inhibitory composition and their use.

The use of olpadronate (3-dimethylamino-1-hydroxypropylidene-1,1-bisphosphonate) and derivatives as inhibitors of calcium crystal growth is also known in principle. U.S. Patent No. 4,054,598 discloses a method with sequestering agents, especially for alkaline earth metal ions, having the formula 1-hydroxy-3-amino-alkane-1,1-bisphosphonic acids, useful for the treatments of disturbances of calcium or phosphate metabolism characterized by abnormal deposition of difficultly soluble calcium salts or the abnormal disolution of hard tissues causing losses of hard bone substance, which cannot be replaced or only by incompletely crystallized tissues, such as Paget's disease, lithiasis, arthritis and others. Spanish Patent No. P910088 discloses pharmaceutical liposome preparations containing bisphosphonates as active compounds. The use of olpadronate as an anabolic agent for the treatment of osteoporosis and other bone metabolism disorders is claimed in the Applicant's earlier European Patent Application No. 94 120 799.5 (unpublished as yet).

U.S. Patent No. 4,927,814 discloses various bisphosphonate derivatives, pharmaceutical compositions and methods of their use. Amongst others 1-amino substituted bisphosphonic acids with specific amino alcohol terminations are mentioned, although no such 1-amino substituted substance is described in more detail or with any test results for its activity.

It is the object of the invention to provide for further useful bisphosphonic acids, in particular in specific medical uses.

### Summary of the Invention:

According to the present invention, there are provided 1-aminoalkyliden-1,1-bisphosphonic acids of formula (B), or any salt thereof: 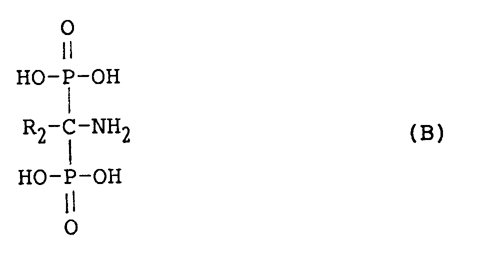 wherein R₂ is a straight-chain or branched aliphatic hydrocarbon radical of 1-9 carbon atoms, which is optionally substituted by one or more amino or aminoalkyl groups, with the exception of a terminal aminoalkyl group 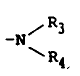 wherein R₃ is a straight-chain or branched, saturated or unsaturated aliphatic hydrocarbon radical of 1-9 carbon atoms, and R₄ is cyclohexyl or cyclohexylmethyl, benzyl or a straight-chain or branched, saturated or unsaturated aliphatic hydrocarbon radical of 4-18 carbon atoms, as a single substituent of R₂.

In particular, the present invention relates to 1-amino-ethyliden-1,1-bisphosphonic acid; 1,3-diaminopropyliden-1,1-bisphosphonic acid; or 3-(N,N-dimethylamino)-1-aminopropyliden-1,1-bisphosphonic acid, or any salt thereof.

Furthermore, the invention relates to a pharmaceutical composition comprising a pharmaceutically effective amount of at least one of the above mentioned bisphosphonic acids or of their monosodium or other pharmaceutically acceptable salt.

Finally, the invention relates to specific methods of use of the compounds according to the present invention.

One of those uses is as a biological carrier for other bone-active substances, for example selected from the group consisting of cytokines, growth factors, prostaglandins, hormones, and cytostatic drugs.

Another kind of use is for the preparation of a medicament for the diagnosis, prophylaxis and/or treatment of bone and/or mineral metabolism disorders, in particular urolithiasis, ectopic calcifications, all forms of osteoporosis, all forms of arthritis and all form of periodontal diseases.

For further improvement of those uses, the invention proposes the simultaneous or sequential administration of at least one calcium salt and/or vitamin D or derivatives thereof and/or at least one fluoride salt and/or at least one parathyroid hormone and/or at least one androgen and/or at least one estrogen.

Bisphosphonates with a hydroxy group in R₁ show high affinity for calcium crystals and this is attributed to the tridentate binding to the crystal surfaces. It has now been discovered by the inventors hereof that surprisingly tridentate binding can equivalently be achived by substituting the hydroxyl group with an amino group. The hydroxyl group in R₁ in the structure of three clinically useful bisphosphonates, namely etidronate (1-hydroxyethylidene-1,1-bisphosphonate), pamidronate (3-amino-1-hydroxypropyliden-1,1-bisphosphonate) and olpadronate (3-dimethylamino-1-hydroxypropyliden-1,1-bisphosphonate) was substituted by an amino group resulting in the following compounds: 1-aminoethylidene-1,1-bisphosphonic acid, 1,3-diaminopropylidene-1,1-bisphosphonic acid, and 3-dimethylamino-1-aminopropylidene-1,1-bisphosphonic acid, respectively.

Etidronate and pamidronate were obtained by a process known from Argentine Patent No. 200,473 that discloses a process for the preparation of 1-hydroxyalkyliden-bisphosphonic acids and their salts, and Argentine Patent No. 218,558 that discloses a process to prepare 3-amino-1-hydroxypropylidene-bisphosphonic acids and their salts. Olpadronate is obtained by a process described in above-mentioned, unpublished European Patent Application No. 94 120 799.5. The novel compounds, i.e. the R₁-amino substituted bisphosphonic acids, were obtained by a synthesis process described hereinbelow (see Examples 1 to 3).

The new bisphosphonates with an amino substitution at R₁ exhibited physicochemical properties (binding to bone mineral, inhibition of calcium incorporation to bone, inhibition of crystal growth) comparable to their respective hydroxyl analogs (see Examples 4 to 7).

3-dimethylamino-1-aminopropylidene-1,1-bisphosphonic acid (referred to as compound X) was, however, additionally devoid of any antiresorptive activity. The amino substitution affected also to some extent the antiresorptive potency of the pamidronate analog, but not of the etidronate analog.

At least two of the new compounds (compound X and compound VII) can therefore be used in the treatment of conditions in which a potent antiresorptive action is unwanted while targeting to calcium crystals and/or retention of other properties is required. Examples include the diagnosis, prophylaxis and/or treatment of urolithiasis, ectopic calcifications, their use as specific carriers for other bone active molecules (including, but not restricted to, cytokines, growth factors, prostaglandins, hormones, etc.) or cytostatic drugs to the skeleton, either for diagnosis or therapeutic purposes, or when the specific properties of the R₂ group on bone metabolism should be retained (e.g. anabolic effect) such as in the treatment of all forms of osteoporosis, all forms of arthritis and periodontal diseases.

However, the other novel compounds may also be useful in the methods of use as mentioned hereabove.

The novel active substances may be used in oral formulations or administered introveneously with or without a pharmaceutical acceptable carrier, diluent or vehicle.

### Description of the Drawings:

Fig. 1 shows the results of the binding tests of various known and novel compounds to bone mineral;
Fig. 3 shows the inhibition of bone resorption of the same compounds.

### Detailed Description of the Invention:

### Example 1

### Synthesis of 1-amino-ethyliden-1,1-bisphosphonic acid (compound III) and monosodium salt (compound IV)

a. Mix 84 ml of water with 150 ml of phosphorus trichloride. Phosphorus acid (I) is formed.
b. Heat I to 130°C and add 20 ml of acetonitrile. Maintain the temperature during 12 hours.
c. Cool to room temperature and add 250 ml of methanol.
d. Cool to 0-5°C, filter, wash with methanol and dry. The yield is 21.2 g (26%) of a colorless product which melts at 263°C with decomposition (II).
e. Suspend the product in 43 ml of water. Add a solution of 4.4 g of sodium hydroxide in 15 ml of water and heat to 60°C. At this point, the product dissolves and a crystalline colorless precipitate is formed. Cool, filter, wash with water and dry. The yield is 34.9 g of the monosodium salt (IV).
f. To convert IV in the acid form (III), suspend 10.2 g of the salt in 90 ml of water. Add 4.2 g of sodium hydroxide and heat to 70°C until total dissolution. Add concentrated hydrochloric acid until pH = 1 (approximately 10 ml), cool, filter and wash with cold water. The yield is 9.34 g (100%) of colorless crystals which melt at 254-255°C with decomposition.

### COMPOUND III

### 1-amino-ethyliden-1,1-bisphosphonic acid

- **MOLECULAR FORMULA:**: C₂H₉NO₆P₂
- **MOLECULAR WEIGHT:**: 205,995

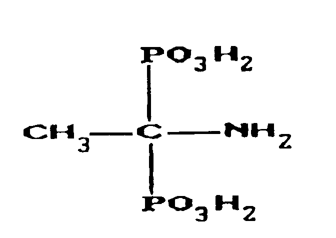

| **ELEMENTAL ANALYSIS:** | | | |
|---|---|---|---|
| Found: | C: 13,33% | H: 4,73% | N: 6,78% |
| Calculated: | C: 11,66% | H: 4,89% | N: 6,80% |

### SPECTRAL PROPERTIES:

- ^{**1**}**H-NMR:**:
- Solvent:: D₂O/D₂SO₄
- Equipment:: BRUCKER AC-200

| Chemical Shifts (ppm) | Multiplicity | Number of protons | ASSIGNMENTS |
|---|---|---|---|
| 2,21 | t | 3 | CH₃ ³J_{H-P}= 14,5 Hz |

### Example 2

### Synthesis of 1,3-diamino-propylidene-1,1-bisphosphonic acid, monosodium salt (compound VII):

a. Add 39 ml of phosphorous tribromide to a suspension of 11.59 g of 3-aminopropionitrile and 8.93 g of phosphorous acid in 80 ml of dioxane at 40°C. Heat to 75-80°C and maintain that temperature during 7 hours.
b. Add 36 ml of water and heat under reflux during 2.5 hours.
c. Cool to 5°C and filter from an orange impurity.
d. Add 125 ml of isopropanol to solid and stir during 15 hours. Filter and dry. The yield is 1.43 g of a colorless solid that by suspension in water and filtration gives 783 mg of a solid which melts at 258-265°C (V).
e. Suspend V in 2.5 ml of water, add 0.244 g of sodium hydroxide, which produces dissolution.
f. Add 7 ml of methanol. A white solid is produced (VI). Filter at 0°C and dissolve in 1.5 ml of water.
g. Add 7 ml of methanol, cool to 5°C, filter and dry at 40°C. The yield is 800 mg of a colorless solid (VII), homogeneous by thin layer chromatography, which does not melt at 320°C.

### COMPOUND VII

### 1,3-diamino-propylidene-1,1-bisphosphonic acid, monosodium salt

- **MOLECULAR FORMULAR:**: C₃H₁₁N₂O₆P₂Na
- **MOLECULAR WEIGHT:**: 256,067

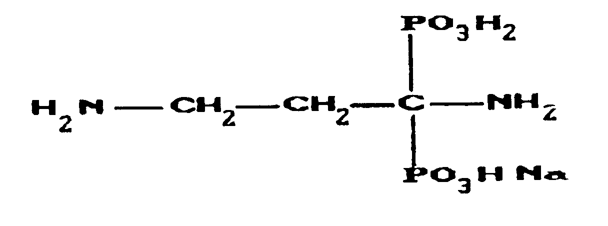

### SPECTRAL PROPERTIES:

- ^{**1**}**H-NMR:**:
- Solvent:: D₂O/D₂SO₄
- Equipment:: BRUCKER AC-200

| Chemical Shifts (ppm) | Multiplicity | Number of protons | ASSIGNMENTS |
|---|---|---|---|
| 2,19 | m | 2 | -CH₂-C-P |
| 3,32 | m | 2 | N-CH₂-C |

### Example 3

### Synthesis of 1-amino-3-(N,N-dimethylamino)-propylidene-1,1-bisphosphonic acid (compound X):

a. Add 174.5 ml of phosphorous trichloride to a solution of 57.5 ml of N,N-dimethylaminopropionitrile in 102 ml of 70% methanesulfonic acid at room temperature.
b. Heat under nitrogen to 65°C during 6 hours.
c. Cool to approximately 25°C, add 200 ml of water, heat under reflux during 5 hours and filter to eliminate a yellow solid in suspension (VIII).
d. Add 1.3 l of isopropanol to the filtrate with stirring. Cool to 0°C and filter.
e. Suspend the solid in isopropanol:water (6:4), filter again and dry. The yield is 28.3 g (21.7%) of a color-less solid (IX).
f. Suspended IX in 56 ml of water. Add a solution of 4.5 g of sodium hydroxide in 30 ml of water, heat to 80°C and filter while hot.
g. Add concentrated hydrochloric acid to the filtrate until pH = 1 (about 13.5 ml), cool and filter.
h. Dissolve the solid in a solution of 3.4 g of sodium hydroxide in 60 ml of water at 65°C.
i. Add concentrated hydrochloric acid to pH = 1, cool to 0°C, filter and dry. The yield is 20.6 g (73%) of colorless crystals which melt at 275°C with decomposition (X).

### COMPOUND X

### 1-amino-3-(N,N-dimethylamino)-propyliden-1,1-bisphosphonic acid

- **MOLECULAR FORMULA:**: C₅H₁₆N₂O₆P₂
- **MOLECULAR WEIGHT:**: 262,148

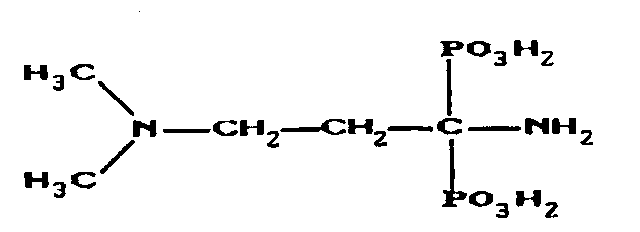

| **ELEMENTAL ANALYSIS:** | | | |
|---|---|---|---|
| Found: | C: 23,44% | H: 7,41% | N: 10,11% |
| Calculated: | C: 22,91% | H: 6,15% | N: 10,69% |

### SPECTRAL PROPERTIES:

- ^{**1**}**H-NMR:**:
- Solvent:: D₂O/D₂SO₄
- Equipment:: BRUCKER AC-200

| Chemical Shifts (ppm) | Multiplicity | Number of protons | ASSIGNMENTS |
|---|---|---|---|
| 9,41 | s | 1 | ⁺N-H |
| 2,65 | bt | 2 | CH₂-N |
| 2,05 | s | 6 | CH₃-N-CH₃ |
| 1,71-1,48 | m | 2 | CH₂-C-P |

### Example 4

### Binding to bone mineral

Binding of compounds (III), (VII) and (X) with an amino group at R₁ to bone was examined by their ability to displace 14C-bisphosphonate from mouse fetal explants according to accepted methodology (van Beek et al, Journal of Bone and Mineral Research, (1994), Vol. 9, p. 1875-1882) and was compared to that of their hydroxyl analogs, the bisphosphonates etidronate, pamidronate and olpadronate.

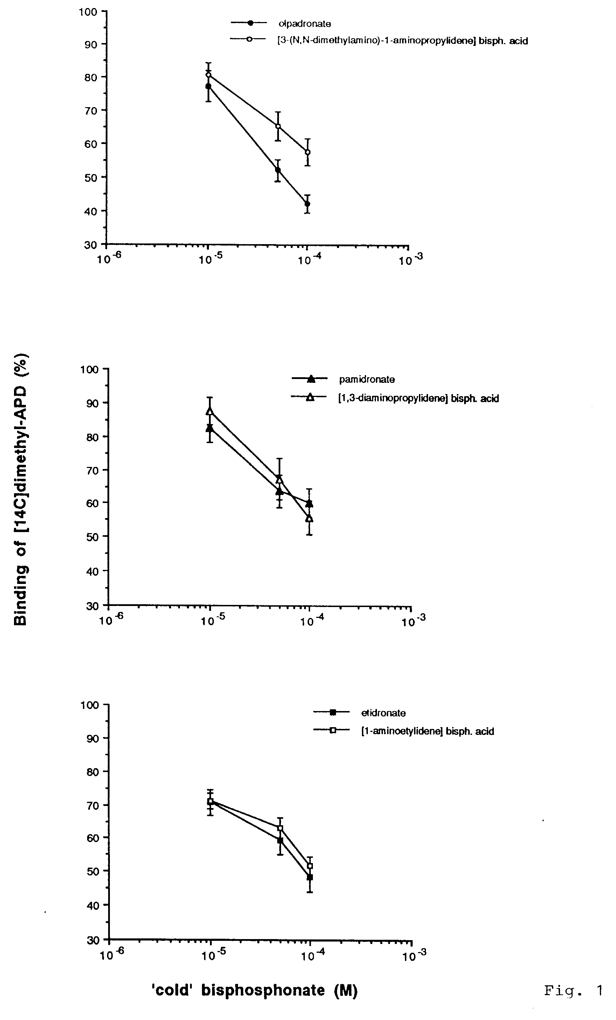

All six bisphosphonates tested bound to the explants dose-dependently. There were no differences between etidronate and compound (III) and pamidronate and compound (VII) and olpadronate and compound (X). The results are shown in Fig. 1.

### Example 5

### Inhibition of 45 calcium incorporation into osteoclast-devoid bones.

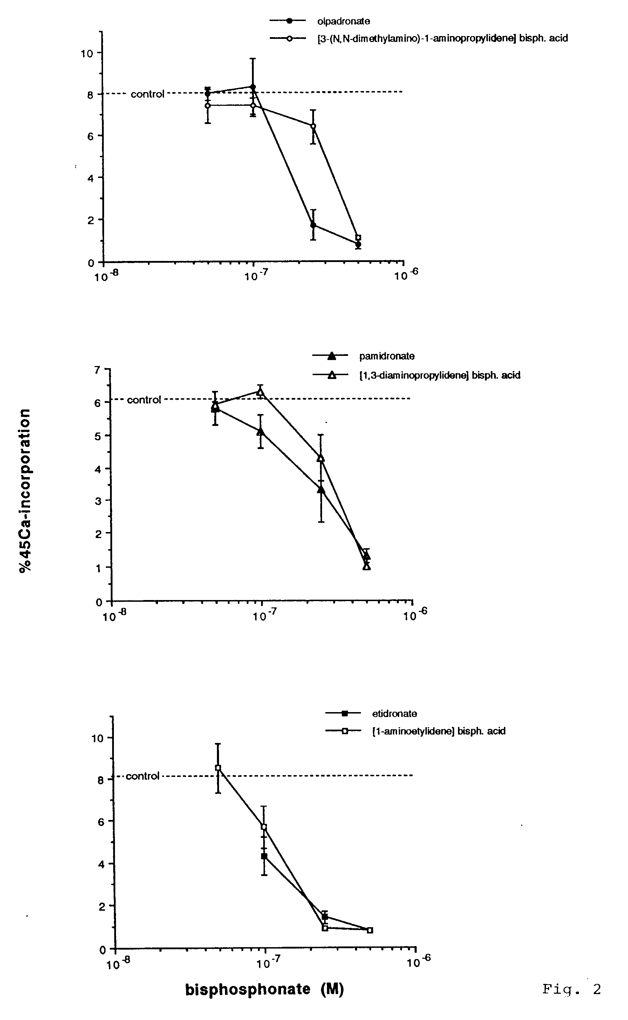

45 calcium incorporation into osteoclast-devoid fetal bones of mice was inhibited by all bisphosphonates dose-dependently. There were no differences between the three hydroxybisphosphonates (etidronate, pamidronate and olpadronate) and their respective aminosustituted analogs (compounds III, VII and X). Half-maximal inhibiting concentrations: etidronate and compound III: 1.5 x 10⁻⁷ M; pamidronate and compound VII: 2 x 10⁻⁷ and 2.5 x 10⁻⁷ M, respectively; olpadronate and compound X: 2 x 10⁻⁷ and 4 x 10⁻⁷ M, respectively. The results are shown in Fig. 2.

### Example 6

### Inhibition of crystal growth.

Olpadronate and compound X were also tested (Methode: Kok et al., Kidney Int. 1988, Vol. 34, p. 346-350) for their ability to inhibit the growth of calcium oxalate monohydrate crystals using a seeded crystal growth system. Both compounds inhibited the growth of the calcium crystals roughly equipotently (half-maximal concentrations: 6 x 10⁻⁶ M and 3 x 10⁻⁶ M, respectively).

### Example 7

### Inhibition of bone resorption.

Fetal mouse metacarpal bones prelabelled with 45 calcium were treated with various concentrations of the six bisphosphonates tested in the previous experiments, and were cultured for 10 days. Resorption was assessed as percentage of 45 calcium release relative to control according to standard methodology (Van der Pluijm et al., Endocrinology, (1991), Vol. 129, p. 1596-1604).

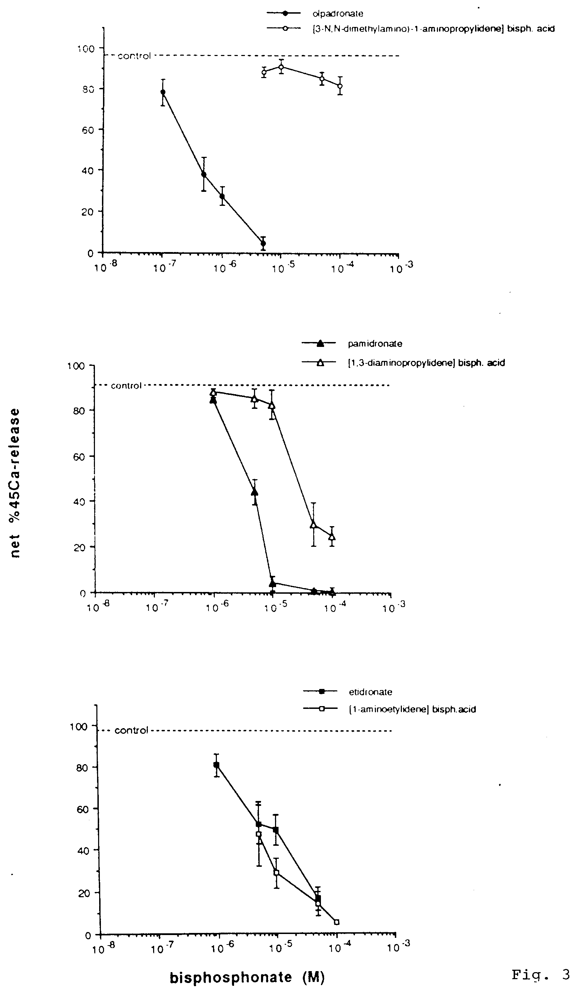

Etidronate and its analogous compound III suppressed 45 calcium release equipotently; pamidronate was about 6 times more potent than compound VII, while compound X showed absolutely no antiresorptive activity in contrast to the increased potency of olpadronate. The results are shown in Fig. 3.

According to previous results (Papapoulos et al., Journal of Bone and Mineral Research, 1989, Vol. 4, p. 775-781) those in vitro data allow prediction of equivalent in vivo effects.

The inventive features disclosed in the preceding description, as well as in the claims and drawings can be essential to the realization of the invention in its various embodiments, either singly or in the form of random combinations.

## Claims

1. 1-Aminoalkyliden-1,1-bisphosphonic acids of formula (B), or any salt thereof: 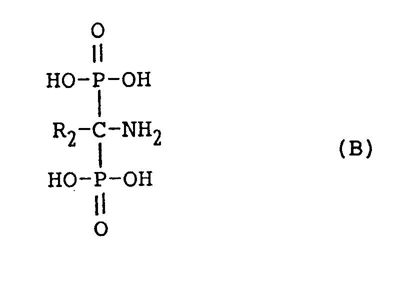 wherein R₂ is a straight-chain or branched aliphatic hydrocarbon radical of 1-9 carbon atoms, which is optionally substituted by one or more amino or aminoalkyl groups with the exception of a terminal aminoalkyl group 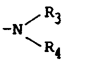 wherein R₃ is a straight-chain or branched, saturated or unsaturated aliphatic hydrocarbon radical of 1-9 carbon atoms, and R₄ is cyclohexyl or cyclohexylmethyl, benzyl or a straight-chain or branched, saturated or unsaturated aliphatic hydrocarbon radical of 4-18 carbon atoms, as a single substituent of R₂.

2. The compound according to claim 1, being 1-aminoethyliden-1,1-bisphosphonic acid; 1,3-diaminopropyliden-1,1-bisphosphonic acid; or 3-(N,N-dimethylamino)-1-aminopropyliden-1,1-bisphosphonic acid, or any salt thereof.

3. A pharmaceutical composition comprising a pharmaceutically effective amount of at least one of bisphosphonic acids of claim 1 or 2 or of their monosodium or other pharmaceutically acceptable salt.

4. Use of one of the bisphosphonic acids of claim 1 or 2 or their monosodium or pharmaceutically acceptable salt, as a biological carrier for other bone-active substances.

5. The use according to claim 4 wherein said other bone-active substance is selected from the group consisting of cytokines, growth factors, prostaglandins, hormones and cytostatic drugs.

6. The use of at least one of the compounds of claim 1 or 2 for the preparation of a medicament for the diagnosis, prophylaxis and/or treatment of bone and/or mineral metabolism disorders.

7. The use according to claim 6 for the preparation of a medicament for the diagnosis, prophylaxis and/or treatment of urolithiasis, ectopic calcifications, all forms of osteoporosis, all forms of arthritis and all forms of periodontal diseases.

8. The use according to one of claims 4-7, comprising the simultaneous or sequential administration of at least one calcium salt.

9. The use according to one of claims 4-8, comprising the simultaneous or sequential administration of vitamin D or derivatives thereof.

10. The use according to one of the preceding claims, comprising the simultaneous or sequential administration of at least one fluoride salt.

11. The use according to one of the preceding claims, comprising the simultaneous or sequential administration of at least one parathyroid hormone.

12. The use according to one of the preceding claims, comprising the simultaneous or sequential administration of at least one androgen.

13. The use according to one of the preceding claims, comprising the simultaneous or sequential administration of at least one estrogen.
